(19) **Europäisches Patentamt / European Patent Office / Office européen des brevets**

(11) **EP 4 775 259 A1**

(12) **EUROPEAN PATENT APPLICATION**

(43) Date of publication:
**15.07.2026 Bulletin 2026/29**

(21) Application number: **26150119.1**

(22) Date of filing: **05.01.2026**

(51) International Patent Classification (IPC):
**A61N 5/10** (2006.01)

(52) Cooperative Patent Classification (CPC):
**A61N 5/103**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **08.01.2025 US 202563742940 P**
**21.08.2025 US 202519305914**

(71) Applicant: **Siemens Healthineers AG**
**91301 Forchheim (DE)**

(72) Inventors:
- **GAO, Riqiang**
  **Princeton, NJ, 08540-6632 (US)**
- **DIALLO, Mamadou**
  **Princeton, NJ, 08540-6632 (US)**
- **ARBERET, Simon**
  **Princeton, NJ, 08540-6632 (US)**
- **KRAUS, Martin**
  **91052 Erlangen (DE)**
- **GHESU, Florin-Cristian**
  **91052 Erlangen (DE)**
- **KAMEN, Ali**
  **Princeton, NJ, 08540-6632 (US)**

(74) Representative: **HKW Intellectual Property PartG mbB**
**Theresienhöhe 12**
**80339 München (DE)**

(54) **AUTOMATING HIGH QUALITY RADIOTHERAPY PLANNING AT SCALE**

(57) Systems and methods for generating radiotherapy plans are provided. One or more medical images are received. One or more anatomical objects are segmented from the one or more medical images. Radiotherapy configurations for one or more objectives are determined based on the one or more medical images and the segmentations of the one or more anatomical objectives. A candidate radiotherapy plan is generated based on the radiotherapy configurations. A quality assessment of the candidate radiotherapy plan is performed. The one or more objectives are updated based on results of the quality assessment and the determining, the generating, and the performing are repeated for one or more iterations using the updated one or more objectives as the radiotherapy objectives to generate the candidate radiotherapy plan for a last iteration as a final radiotherapy plan. The final radiotherapy plan is output. Since the pipeline is automatic, the plan generation can be scaled. The data can serve large-scale AI model training.

FIG. 1

100

Receive one or more medical images
102

Segment one or more anatomical objects from the one or more medical images
104

Determine radiotherapy configurations for one or more objectives based on the one or more medical images and the segmentations of the one or more anatomical objects
106

Generate a candidate radiotherapy plan based on the radiotherapy configurations
108

Perform a quality assessment of the candidate radiotherapy plan
110

Update the one or more objectives based on results of the quality assessment and repeating steps 106, 108, and 110 for one or more iterations using the updated radiotherapy objectives and the radiotherapy objectives to generate the candidate radiotherapy plan for a last iteration as a final radiotherapy plan
112

Output the final radiotherapy plan
114

**Description**

CROSS-REFERENCE TO RELATED APPLICATIONS

This application claims the benefit of U.S. Provisional Application No. 63/742,940, filed January 8, 2025, the disclosure of which is herein incorporated by reference in its entirety.

TECHNICAL FIELD

The present invention relates generally to automatic radiotherapy planning, and in particular to automating high quality radiotherapy planning at scale.

BACKGROUND

Radiotherapy planning is important for ensuring precise and effective cancer treatment. Radiotherapy planning is a complex and highly specialized process that requires close coordination among clinical experts, including radiation oncologists, medical physicists, and dosimetrists. In practice, radiotherapy planning involves CT (computed tomography) scan simulation, manual contouring of PTVs (planning target volumes) and OARs (organs at risk), prescription definition, configuration of planning parameters, initial plan generation, physician review, and iterative refinement. The entire radiotherapy planning process usually involves 3 to 6 hours of active work over 1 to 5 days. Furthermore, the radiotherapy planning process relies heavily on manual input and subjective clinical judgment.

AI (artificial intelligence) has recently been applied to various stages of the radiotherapy planning process, such as dose prediction, fluence prediction, leaf sequencing, and dose calculation. AI based radiotherapy planning has demonstrated potential in improving treatment precision, efficiency, and consistency. However, the variability in radiotherapy planning poses significant challenges for AI model training. Such variability stems from differences in PTV/OAR contouring styles, planning preferences, auxiliary structures, clinical protocols, and human biases. Further compounding this issue, many of the available radiotherapy datasets do not include explicit definitions of optimization objectives, making it difficult to align contoured structures with their corresponding radiotherapy dose distributions.

BRIEF SUMMARY OF THE INVENTION

The invention is defined by the attached set of claims. Further details of the disclosed methods, devices and systems are described below, which are helpful for understanding the claimed invention.

In accordance with one or more embodiments, systems and methods for generating radiotherapy plans are provided. One or more medical images are received. One or more anatomical objects are segmented from the one or more medical images. Radiotherapy configurations for one or more objectives are determined based on the one or more medical images and the segmentations of the one or more anatomical objectives. A candidate radiotherapy plan is generated based on the radiotherapy configurations. A quality assessment of the candidate radiotherapy plan is performed. The one or more objectives are updated based on results of the quality assessment and the determining, the generating, and the performing are repeated for one or more iterations using the updated one or more objectives as the one or more objectives to generate the candidate radiotherapy plan for a last iteration as a final radiotherapy plan. The final radiotherapy plan is output.

In one embodiment, a score is calculated for each of a plurality of metrics for the candidate radiotherapy plan. The scores are combined to generate a final score of the candidate radiotherapy plan.

In one embodiment, the results of the quality assessment are compared with results of a quality assessment of a preceding iteration. It is determined whether the comparison satisfies a threshold. In response to determining that the comparison does not satisfy the threshold, the determining, the generating, and the performing are repeated.

In one embodiment, one or more metrics resulting from the quality assessment of the candidate radiotherapy plan are added to the one or more objectives.

In one embodiment, a margin is added to at least one of the one or more objectives based on the results of the quality assessment of the candidate radiotherapy plan.

In one embodiment, PTV (planning target view) helper structures are generated based on the one or more medical images. The radiotherapy configurations for the one or more objectives are determined further based on the PTV helpers.

In one embodiment, the radiotherapy configurations comprise beam configuration and dose.

In one embodiment, the radiotherapy configurations comprise a maximum and minimum dose and dose volume constraints.

In one embodiment, an AI (artificial intelligence) model is trained based on the final radiotherapy plan.

These and other advantages of the invention will be apparent to those of ordinary skill in the art by reference to the following detailed description and the accompanying drawings.

BRIEF DESCRIPTION OF THE DRAWINGS

Figure 1 shows a method for automatically generating radiotherapy plans, in accordance with one or more embodiments;

Figure 2 shows a workflow for automatically generating radiotherapy plans, in accordance with one or more embodiments;

Figure 3 shows a table of exemplary OARs (organs at risk) and PTV (planning target volume) helper structures utilized head-and-neck cancer in accordance with one or more embodiments;

Figure 4 shows a table 400 of objectives for lung cancer treatment, in accordance with one or more embodiments;

Figure 5 shows a table of exemplary quality assessments of a candidate radiotherapy plan for head-and-neck cancer, in accordance with one or more embodiments;

Figure 6 shows a table of exemplary quality assessments of a candidate radiotherapy plan for lung cancer, in accordance with one or more embodiments;

Figure 7 shows exemplary treatment plans, in accordance with one or more embodiments;

Figure 8 shows a workflow for iteratively generating a radiotherapy plan, in accordance with one or more embodiments;

Figure 9 shows an algorithm for automatically generating radiotherapy plans, in accordance with one or more embodiments;

Figure 10 shows an algorithm for calculating a margin, in accordance with one or more embodiments;

Figure 11 shows an algorithm for calculating an objective dose, in accordance with one or more embodiments;

Figure 12 shows a data curation pipeline, in accordance with one or more embodiments;

Figure 13 shows graphs depicting quality improvement achieved through iterative refinement across head-and-neck cohorts, in accordance with one or more embodiments;

Figure 14 shows an exemplary artificial neural network that may be used to implement one or more embodiments;

Figure 15 shows a convolutional neural network that may be used to implement one or more embodiments;

Figure 16 shows a data flow diagram for using a generative adversarial network, in accordance with one or more embodiments;

Figure 17 shows a schematic structure of a recurrent machine learning model that may be used to implement one or more embodiments; and

Figure 18 shows a high-level block diagram of a computer that may be used to implement one or more embodiments.

DETAILED DESCRIPTION

The present invention generally relates to methods and systems for automating high quality radiotherapy planning at scale. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, it is to be understood that embodiments of the present invention may be performed within a computer system using data stored within the computer system. Further, reference herein to pixels of an image may refer equally to voxels of an image and vice versa.

Embodiments described herein provide for a fully automated iterative radiotherapy planning system capable of generating substantial volumes of consistently high-quality treatment plans for, e.g., training robust, generalized AI (artificial intelligence) models. The fully automated iterative radiotherapy planning system adheres to clinical guidelines and automates many steps such as, e.g., OAR (organ at risk) contouring, helper structure creation, beam setup, optimization, and plan quality assessment. Clinical PTVs (planning target volumes) and prescriptions are also preserved to maintain clinical intent. Advantageously, the fully automated iterative radiotherapy planning system significantly reduces the time for generating radiotherapy plans from the typical 3 to 6 hours to 0.3 to 1 hour, enabling the generation of large-scale, high-quality radiotherapy plans for training AI models. The fully automated iterative radiotherapy planning system also provides for an iterative refinement strategy, enhancing plan quality without requiring human intervention.

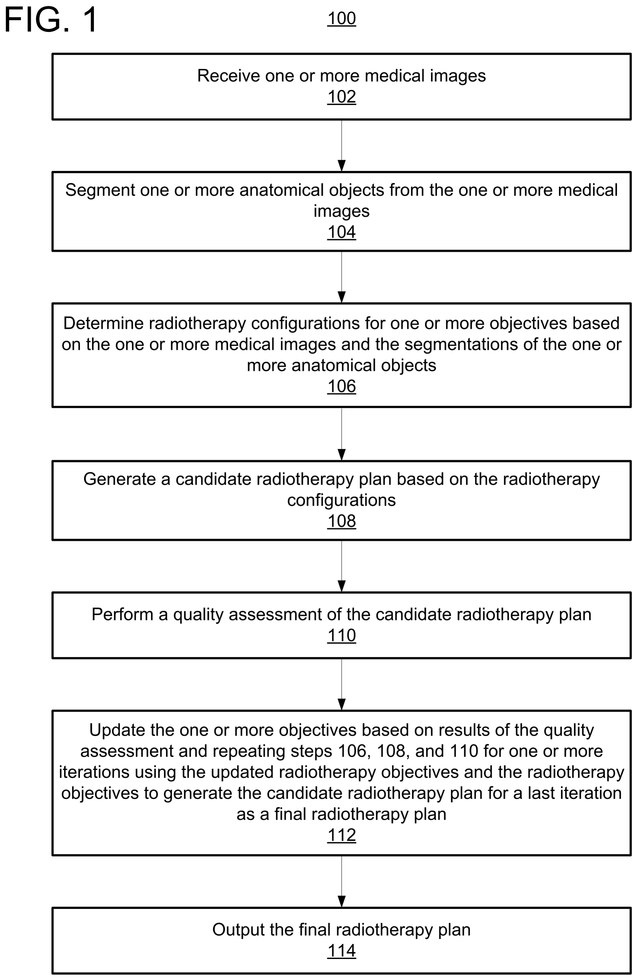

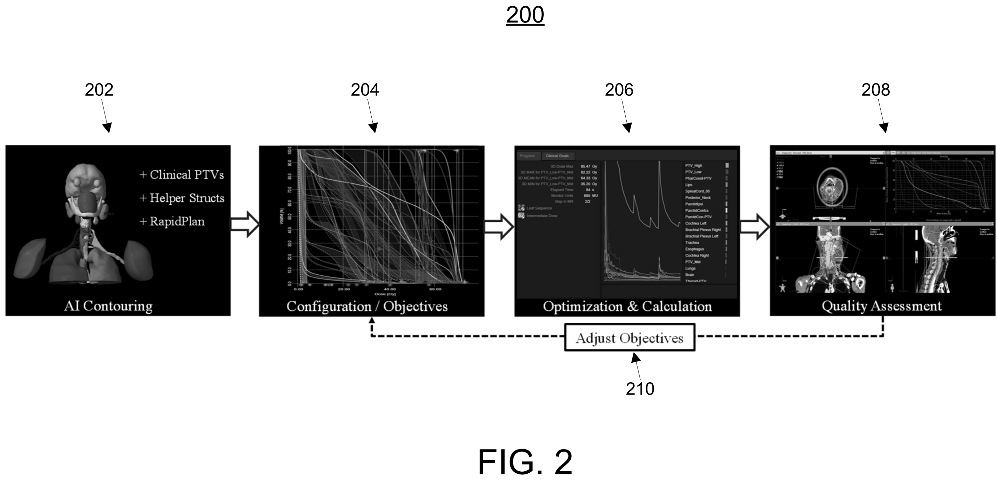

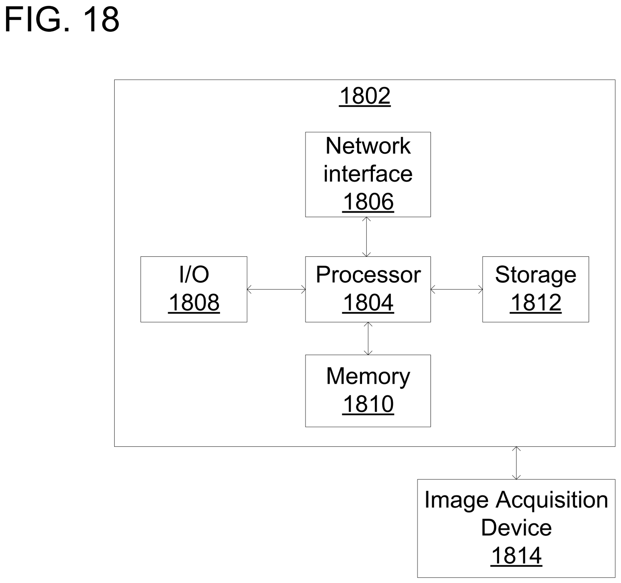

Figure 1 shows a method 100 for automatically generating radiotherapy plans, in accordance with one or more embodiments. The steps and sub-steps of method 100 may be performed by one or more suitable computing devices, such as, e.g., computer 1802 of Figure 18. Figure 2 shows a workflow 200 for automatically generating radiotherapy plans, in accordance with one or more embodiments. Figure 1 and Figure 2 will be described together.

At step 102 of Figure 1, one or more input medical images are received. The one or more medical images depict anatomical objects of a patient relevant for radiotherapy planning. For example, the anatomical objects may comprise organs, bones, vessels, tumors or other abnormalities, or any other anatomical object of interest of the patient.

In one embodiment, the one or more medical images comprise CT (computed tomography) images. However, the one or more medical images may comprise images of any other suitable modality, such as, e.g., MRI (magnetic resonance imaging), US (ultrasound), x-ray, or any other medical imaging modality or combinations of medical imaging modalities. The one or more medical images may be 2D (two dimensional) images and/or 3D (three dimensional) volumes, and may comprise a single medical image or a plurality of medical images.

The one or more medical images may be received, for example, by directly receiving the one or more medical images from the image acquisition device (e.g., image acquisition device 1814 of Figure 18) as the images are acquired, by loading the one or more medical images from a storage or memory of a computer system (e.g., storage 1812 or memory 1810 of computer 1802 of Figure 18), or by receiving the one or more medical images from a remote computer system (e.g., computer 1802 of Figure 18). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, one or more anatomical objects are segmented from the one or more medical images. In one example, as shown in workflow 200 of Figure 2, one or more anatomical objects are segmented at AI contouring step 202.

In one embodiment, the one or more anatomical objects that are segmented from the one or more medical images are predefined anatomical objects (e.g., OARs) relevant for radiotherapy planning. For example, the one or more anatomical objects may be predefined in a DICOM (digital imaging and communications in medicine) RTSTRUCT (radiotherapy structure sets) file.

The one or more anatomical objects may be segmented according to any suitable (e.g., well-known) approach. In one embodiment, the one or more anatomical objects are segmented using a machine learning based segmentation network. The machine learning based segmentation network receives as input the one or more medical images and generates as output segmentation results. The segmentation results may be represented in any suitable form. For example, the segmentation results may be represented as one or more segmentation maps (e.g., binary masks) where each pixel or voxel is assigned to a particular class. In another example, the segmentation results may be represented as contours.

In one embodiment, PTV helper structures are also generated based on the one or more medical images. PTV helper structures are auxiliary structures used in radiotherapy planning to help shape the dose distribution more accurately and protect OARs. The PTV helper structures may comprise, for example, PTV minus OARs (PTV with nearby OARs subtracted) or ring structures (concentric shells around the PTV). The PTV helper structures may be generated using any suitable (e.g., well-known) approach. For example, the PTV helper structures may be generated using Boolean operations (e.g., union, subtraction, intersection) and geometric manipulations performed by a treatment planning system.

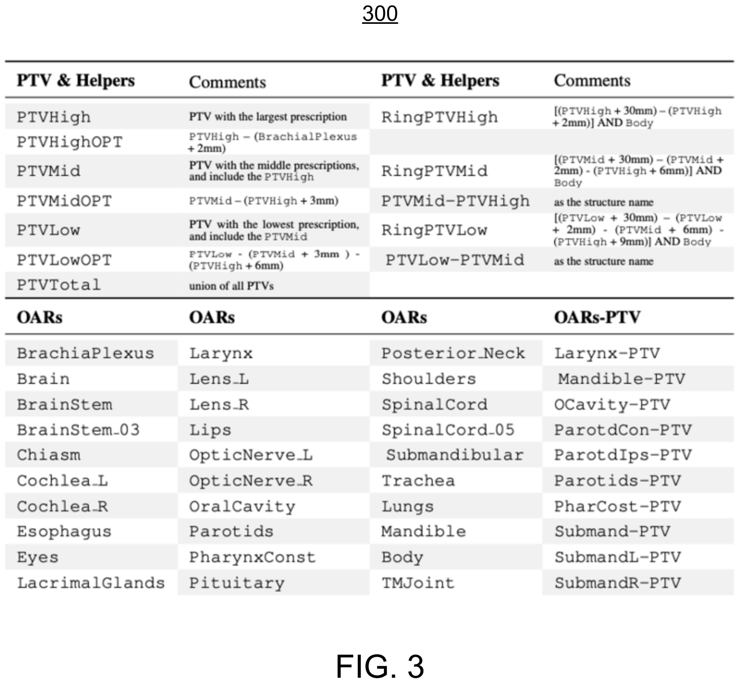

Figure 3 shows a table 300 of exemplary OARs and PTV helper structures utilized head-and-neck cancer in accordance with one or more embodiments. The OARs may be anatomical objects segmented from the one or more medical images and PTV helper structures.

Referring back to Figure 1, at step 106, radiotherapy configurations for one or more objectives are determined based on the one or more medical images and the segmentations of the one or more anatomical objects. For example, as shown in workflow 200 of Figure 2, the radiotherapy configurations for radiotherapy objects are determined at configuration/objectives step 204. In one embodiment, where PTV helpers are also generated, the radiotherapy configurations are determined further based on the PTV helpers.

The one or more objectives are constraints on the radiotherapy configuration to guide radiotherapy treatment. For example, the one or more objectives may comprise a maximum/minimum dose, dose volume constraints, etc. Examples of the radiotherapy configuration include beam configuration (e.g., number of fractions, beam geometries, machine parameters) and dose calculation. In one embodiment, the radiotherapy configurations for the one or more objectives are determined by calling, for example, a beam configuration module, a planning objectives module, and a dose calculation module of ES (Eclipse scripting) API (application programming interface). However, the radiotherapy configuration for the one or more objectives may be determined using any other suitable (e.g., well-known) approach.

In one example, for head-and-neck cancer treatment, four arcs are used for VMAT (volumetric modulated arc therapy), alternating gantry rotations (clockwise and counter clockwise) and setting collimator positions at 30 degrees and 330 degrees. For IMRT (intensity-modulated radiation therapy), two plans per patient are created using 9 and 15 evenly spaced angles. The objectives of the initial plan are primarily defined based on the RapidPlan model, following structure mapping.

In another example, for lung cancer treatment, three different angle ranges are used based on the tumor's location, measured by the lateral distance from the isocenter to the patient's mid-sagittal plane. If the distance exceeds, e.g., 5 centimeters, either the left or right lung template is selected depending on the tumor's side. If the distance is , e.g., 5 centimeters or less, angles are selected that cover the full range. For IMRT, 7 fields are used (if lateral) or 9 angles. For VMAT, two arcs are used.

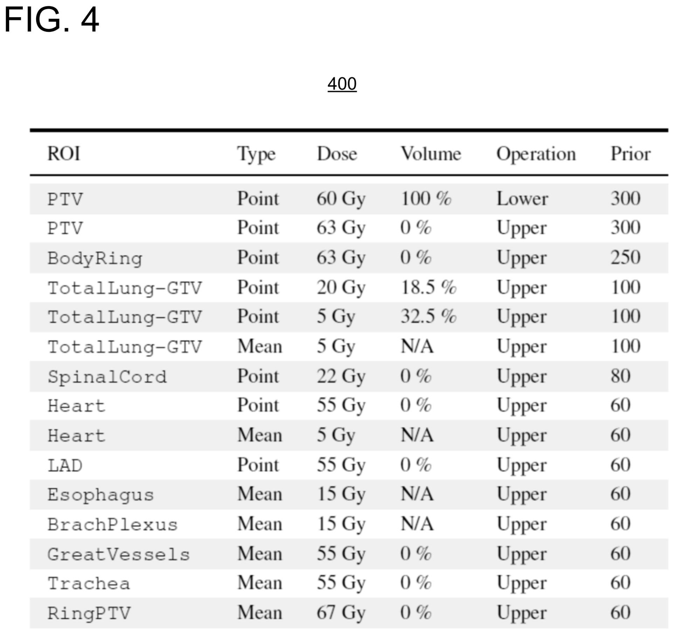

Figure 4 shows a table 400 of objectives for lung cancer treatment, in accordance with one or more embodiments. The "Point" Type represents a point on DVHs (dose-volume histograms), where dose is represented as the x-axis and volume is represented on the y-axis. The "Mean" type represents MeanDose objectives. The "Lower" and "Upper" of the operation column represent lower bounds and upper bounds respectively.

At step 108 of Figure 1, a candidate radiotherapy plan is generated based on the radiotherapy configurations. In one example, as shown in workflow 200 of Figure 2, the candidate radiotherapy plan is generated at optimization and calculation step 206.

The candidate radiotherapy plan may define, for example, one or more angles, beam geometries, and dose. The candidate radiotherapy plan may be generated for IMRT, VMAT, or any other radiotherapy technique. In one example, the candidate radiotherapy plan is generated using RapidPlan (e.g., for head-and-neck cases). In another example, the candidate radiotherapy plan is generated by applying mean/point objectives derived from scorecard metrics (e.g., for lung cases). However, the candidate radiotherapy plan may be generated using any other suitable (e.g., well-known) approach.

At step 110 of Figure 1, a quality assessment of the candidate radiotherapy plan is performed. In one example, as shown in workflow 200 of Figure 2, the quality assessment is performed at step 208.

In one embodiment, the quality assessment is performed by calculating one or more metrics on the candidate radiotherapy plan. The one or more metrics may comprise, for example, volume at dose (VolAtDose), dose at volume (DoseAtVol), mean dose (MeanDose), a DVH relating radiation dose to tissue volume, etc. The one or more metrics may be calculated by a scorecard module in the RapidPlan treatment planning system. In one embodiment, a score is calculated for each of a plurality of metrics and the scores are combined to generate a final score of the candidate radiotherapy plan. The quality assessment may be performed according to any other suitable (e.g., well-known) approach.

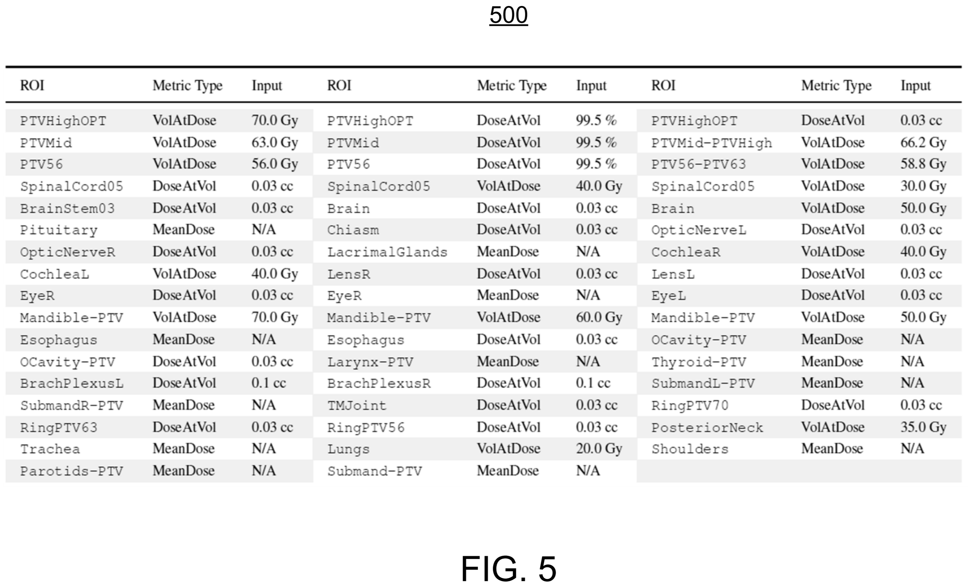

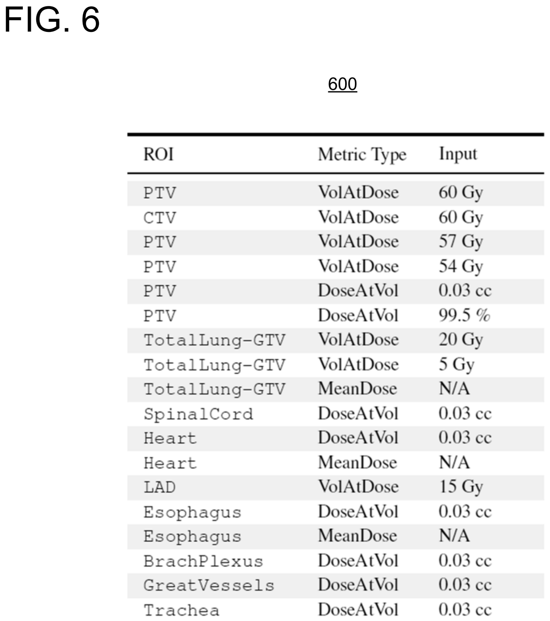

Figure 5 shows a table 500 of exemplary quality assessments of a candidate radiotherapy plan for head-and-neck cancer, in accordance with one or more embodiments. Figure 6 shows a table 600 of exemplary quality assessments of a candidate radiotherapy plan for lung cancer, in accordance with one or more embodiments. Tables 500 and 600 show metrics for VolAtDose, DoseAtVol, and MeanDose are calculated for various ROIs (regions of interest) in proximity to the head-and-neck and the lungs, respectively.

At step 112 of Figure 1, the one or more objectives are updated based on results of the quality assessment and the determining (step 106), the generating (step 108) and the performing (step 110) are repeated for one or more iterations using the updated radiotherapy objectives as the radiotherapy objectives to generate the candidate radiotherapy plan for a last iteration as a final radiotherapy plan. In one example, as shown in workflow 200, the one or more objectives are updated at adjust objectives step 210 and workflow 200 returns to step 204.

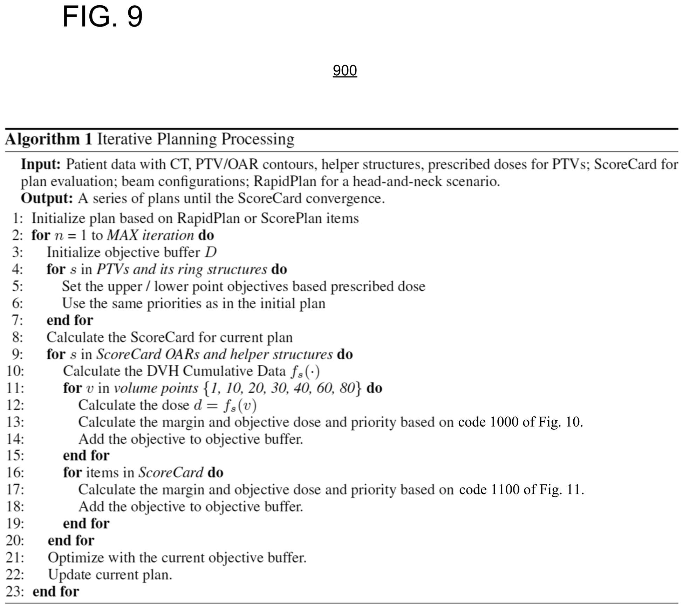

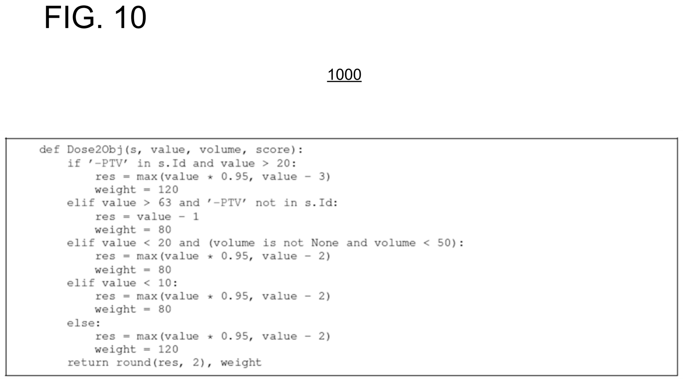

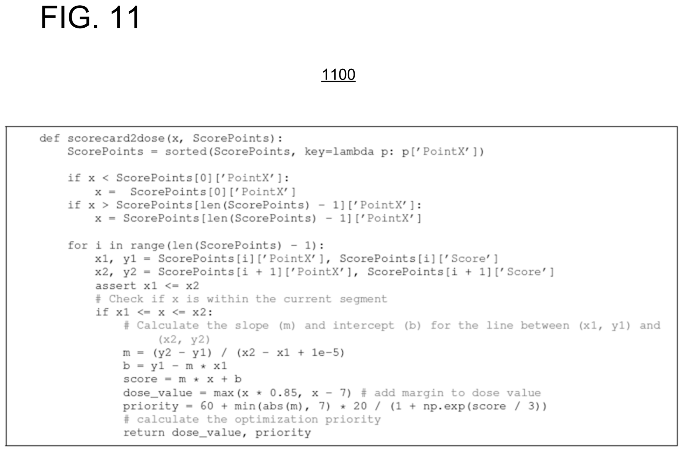

In one embodiment, the upper and lower point objectives may be set based on the prescribed dose, e.g., according to algorithm 900 of Figure 9. In another embodiment, the radiotherapy objectives are updated by adding at least one of the one or more metrics to the one or more objectives and adding some margin. For example, a scorecard may be calculated for the candidate radiotherapy plan and a margin and objective dose and priority may be calculated based on the scorecard, e.g., according to algorithm 900 of Figure 9 and code 1100 of Figure 11. In another embodiment, the radiotherapy objectives are updated by extracting points from the DVH based on the candidate radiotherapy plan and add a margin to the objectives. For example, a dose may be calculated from the DVH and the margin and objective dose and priority may be calculated based on the dose, e.g., according to algorithm 900 of Figure 9 and code 1000 of Figure 10.

The repeating is iteratively performed for one or more iterations. In one embodiment, the repeating may be performed to iteratively generate a candidate radiotherapy plan until the generation of the candidate radiotherapy plan converges. For example, the results (e.g., score) of the quality assessment of the candidate radiotherapy plan and the results (e.g., score) of the quality assessment of the candidate radiotherapy plan for the preceding iteration may be compared and it is determined whether the comparison satisfies (e.g., is less than) a threshold. In response to determining that the comparison does not satisfy the threshold, the determining, the generating, and the performing are repeated. In response to determining that the comparison satisfies the threshold, method 100 proceeds to step 114 of Figure 1. In other embodiments, the repeating may be iteratively performed for a predetermined or maximum number of iterations.

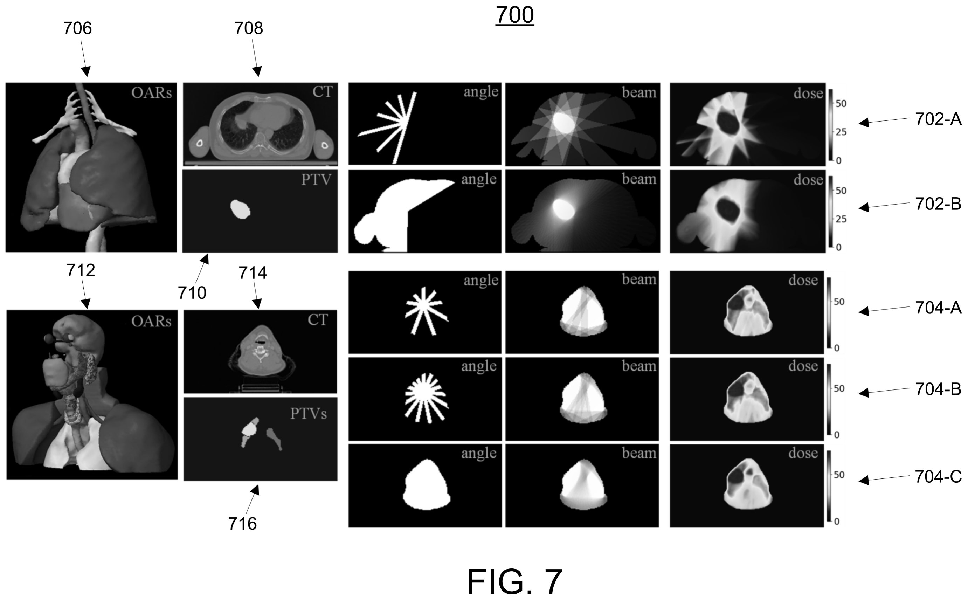

At step 114 of Figure 1, the final radiotherapy plan is output. For example, the final radiotherapy plan can be output by displaying the final radiotherapy plan on a display device of a computer system (e.g., I/O 1808 of computer 1802 of Figure 18), storing the final radiotherapy plan on a memory or storage of a computer system (e.g., memory 1810 or storage 1812 of computer 1802 of Figure 18), or by transmitting the final radiotherapy plan to a remote computer system (e.g., computer 1802 of Figure 18). Exemplary final radiotherapy treatment plans are shown in Figure 7. In one embodiment, the final radiotherapy plan is used to train an AI model for generating a radiotherapy plan for a patient.

Figure 7 shows exemplary treatment plans 700, in accordance with one or more embodiments. Treatment plans 700 comprise treatment plans 702-A and 702-B (collectively referred to as treatment plans 702) for lung cancer and treatment plans 704-A, 704-B, and 704-C (collectively referred to as treatment plans 704) for head-and-neck cancer. Treatment plan 702-A is for IMRT and treatment plan 702-B is for VMAT. Treatment plans 702 are generated based on segmented OARs 706, CT medical images 708, and PTV 710. Treatment plans 704-A and 704-B are for IMRT and treatment plan 704-C is for VMAT. Treatment plans 704 are generated based on segmented OARs 712, CT medical images 714, and PTV 716. Treatment plans 702 and 704 comprise angle, beam, and dose.

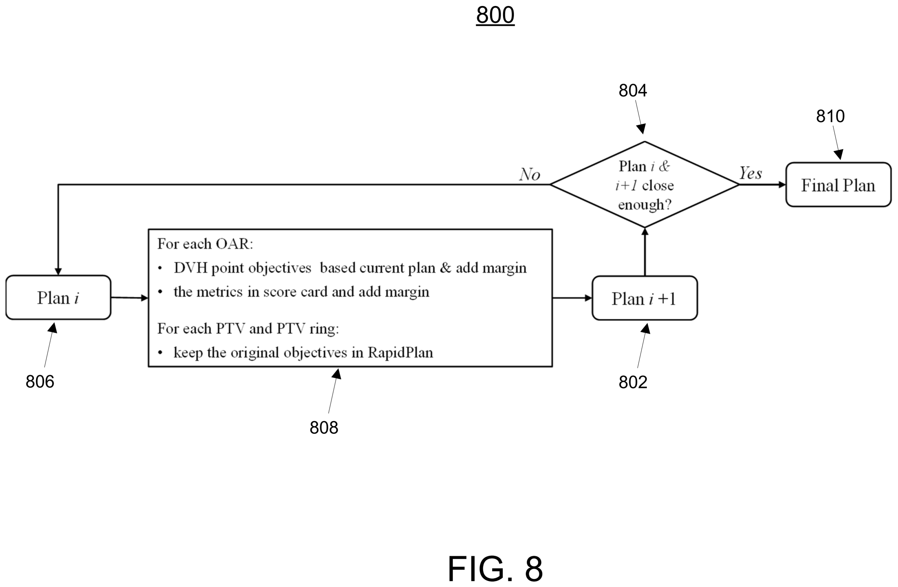

Figure 8 shows a workflow 800 for iteratively generating a radiotherapy plan, in accordance with one or more embodiments. At step 802, a candidate radiotherapy plan i+1 is generated. Step 802 corresponds to step 108 of Figure 1. At step 804, it is determined whether the candidate radiotherapy plan i+1 and the candidate radiotherapy plan i generated during the preceding iteration are close enough. Step 804 corresponds to step 112 of Figure 1. If the candidate radiotherapy plan i+1 and the candidate radiotherapy plan i are not close enough at step 804, workflow 800 returns to step 806 using the candidate radiotherapy plan i+1 as the candidate radiotherapy plan i. At step 808, the radiotherapy objectives are updated. For each OAR, DVH point objectives are updated based on the current candidate radiotherapy plan i and a margin is added. Additionally, metrics in the scorecard are added to the one or more objectives and a margin in added. For each PTV and PTV ring, the original objectives are retained. Workflow 800 proceeds to step 802 to generate candidate radiotherapy plan i+1 based on the updated objectives. If the candidate radiotherapy plan i+1 and the candidate radiotherapy plan i are close enough at step 804, the candidate radiotherapy plan i+1 is output as the final radiotherapy plan at step 810.

Figure 9 shows an algorithm 900 for automatically generating radiotherapy plans, in accordance with one or more embodiments. Algorithm 900 corresponds to the steps of method 100 of Figure 1 and workflow 200 of Figure 2. Algorithm 900 calculates a margin and objective dose according to code 1000 of Figure 10 and code 1100 of Figure 11, according to one or more embodiments.

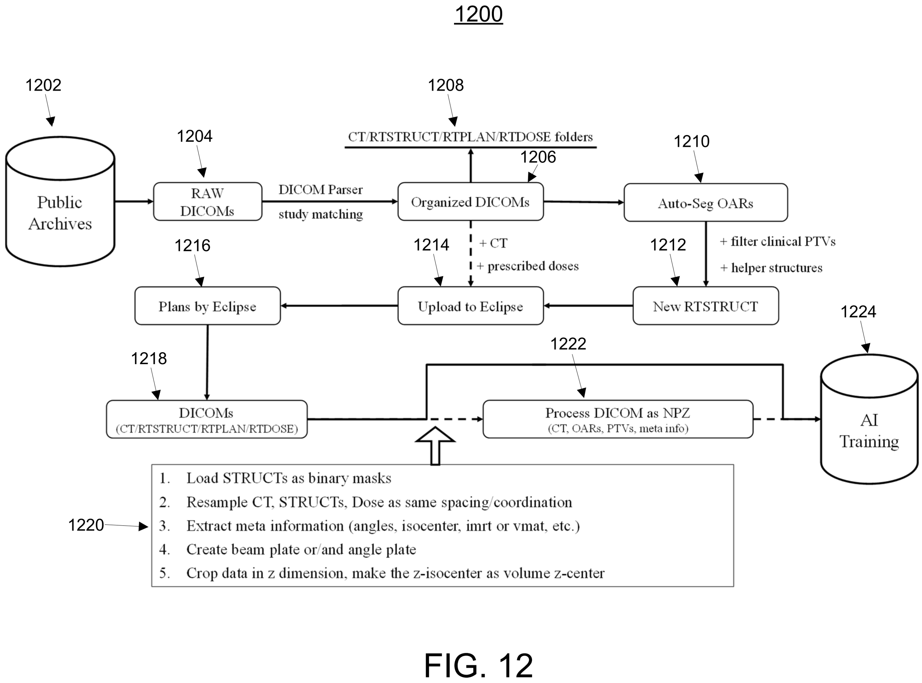

Figure 12 shows a data curation pipeline 1200, in accordance with one or more embodiments. Raw DICOMs 1204 from public archives 1202 or clinical collaborators are typically not well organized. A DICOM parser tool is utilized to structure raw DICOMs 1204 into organized DICOMs 1206 and save organized DICOMs 1206 into separate folders 1208 comprising CT, RTSTRUCT, RTPLAN (if available), and RTDOSE (if available). All OARs are segmented (corresponding to step 104 of Figure 1) at step 1210 from organized DICOMs 1206, filter clinical PTVs through name and dose-level matching, and create helper structures, which are saved as new RTSTRUCT 1212. Along with CT series, new RTSTRUCT 1212 are batch updated to Eclipse at step 1214. Radiotherapy plans are generated at scale at step 1216 (corresponding to steps 106-114 of Figure 1). DICOMs 1218 for CT, RTSTRUCT, RTPLAN, and RTDOSE are exported locally. Exported DICOMs, processed at step 1220, and may be directly used for AI model training 1224. Alternatively, users may process data at step 1222 and save them in more efficient formats (e.g., Numphy) to facilitate data loading.

Embodiments described herein were experimentally validated. Radiotherapy structure creation involves auto-contouring of OARs and creating helper structures, primarily based on clinical PTVs. The auto-contouring part utilizes the latest version of AI-Rad Companion by Siemens Healthineers. To accelerate this process, a C++ executable was developed that generates all necessary structures with a naming convention and exports the results in DICOM format.

To run auto-planning at scale, five GPU (graphics processing unit) computing nodes were built in Microsoft Azure, in which instances of Eclipse 18.0.1 are installed. Each node had 64 GB (gigabyte) of RAM (random-access memory). PyESAPI (python environment for scripting APIs) and Python were used to import DICOM input data and run the scripted algorithm to interact with the Eclipse optimization engine. PyESAPI is a Python-based tool that enables rapid prototyping of C# based ES API built-in functionalities. CT images, PTVs, and prescribed dose information extracted from clinical data was utilized. The public TCIA (the cancer imaging archive) dataset used in this study.

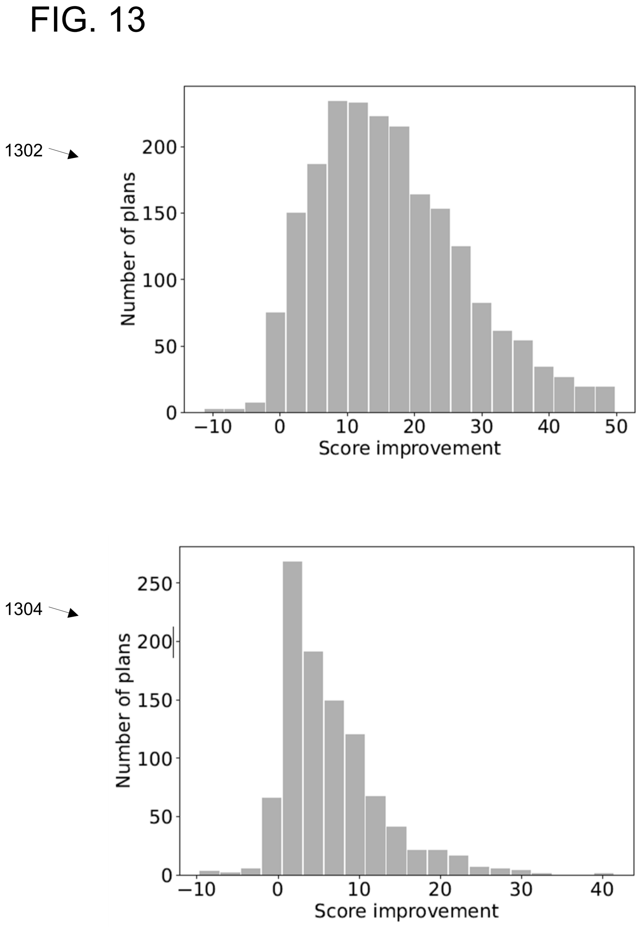

A large number of radiotherapy plans with varying parameters were generated in accordance with method 100 of Figure 1. Figure 13 shows graphs depicting quality improvement achieved through iterative refinement across head-and-neck cohorts, in accordance with one or more embodiments. Positive score improvements represent better plan quality. Graph 1302 shows score improvement of IMRT plans and graph 1304 shows score improvement of VMAT plans. Graphs 1302 and 1304 demonstrate that the iterative refinement in accordance with embodiments described herein improves the scorecards for lung and for head-and-neck treatments, where the modification is that large penalties (instead of 0s in the original score card) are included when criteria are not met.

Comparison with Clinical Plans: Two head-and-neck cases from different cohorts were randomly selected. One notable observation is that the radiotherapy plan generated in accordance with embodiments described herein demonstrates improved PTV homogeneity without compromising OAR sparing. It was observed that the quality of the radiotherapy plan generated in accordance with embodiments described herein is high, even compared to the randomly selected clinical plans. The embodiments described herein generate high quality radiotherapy plans while eliminating human bias, paving the way for unbiased large-scale AI training.

Plan Reproducibility Given 3D Dose: The advantages of plan reproducibility are twofold. First, when a dose prediction model is properly trained, it enables the rapid generation of visualized 3D dose distributions and DVHs based on patient data and beam configuration. This allows users to quickly assess and adjust the treatment plan. For example, a typical head-and-neck case requires approximately 20 minutes for a single forward-planning session, whereas deep learning-based dose prediction methods can generate results in just 0.2 seconds (excluding data loading and preprocessing time). Second, this high reproducibility provides an opportunity to evaluate dose predictions in the context of downstream clinical tasks. In accordance with embodiments described herein, radiotherapy plans were reproduced using only a 3D dose distribution, to thereby evaluate dose prediction.

Embodiments described herein provide for an automated radiotherapy planning pipeline that integrates OAR auto-contouring, scripted radiotherapy configuration and objective setup, scripted optimization and dose calculation, and automated iterative refinement guided by quality evaluation. The first released dataset in generated in accordance with embodiments described herein offered more than 10 times the number of plans compared to the largest existing well-curated public dataset. The released dataset can support a wide range of research topics within the radiotherapy domain. Embodiments described herein represent a significant step toward enabling large-scale AI research in radiotherapy. Notably, the pipeline in accordance with embodiments described herein is designed to be flexible, making it possible to simulate data at scale with sufficient computational resources and time. This pipeline may be a foundational tool for advancing AI applications in radiotherapy and facilitating future research.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., any of the machine learning/artificial intelligence based models disclosed herein, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

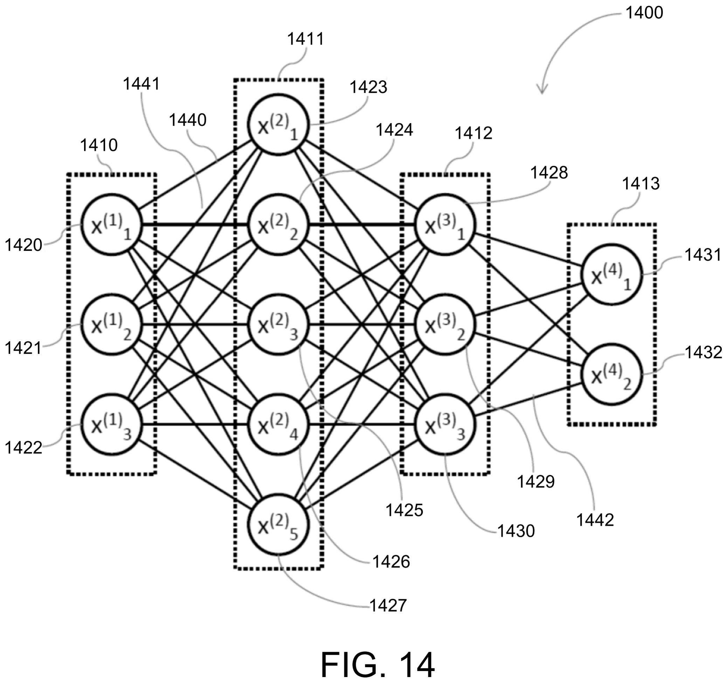

Figure 14 shows an embodiment of an artificial neural network 1400 that may be used to implement one or more machine learning models described herein. Alternative terms for "artificial neural network" are "neural network", "artificial neural net" or "neural net".

The artificial neural network 1400 comprises nodes 1420, ..., 1432 and edges 1440, ..., 1442, wherein each edge 1440, ..., 1442 is a directed connection from a first node 1420, ..., 1432 to a second node 1420, ..., 1432. In general, the first node 1420, ..., 1432 and the second node 1420, ..., 1432 are different nodes 1420, ..., 1432, it is also possible that the first node 1420, ..., 1432 and the second node 1420, ..., 1432 are identical. For example, in Figure 14 the edge 1440 is a directed connection from the node 1420 to the node 1423, and the edge 1442 is a directed connection from the node 1430 to the node 1432. An edge 1440, ..., 1442 from a first node 1420, ..., 1432 to a second node 1420, ..., 1432 is also denoted as "ingoing edge" for the second node 1420, ..., 1432 and as "outgoing edge" for the first node 1420, ..., 1432.

In this embodiment, the nodes 1420, ..., 1432 of the artificial neural network 1400 can be arranged in layers 1410, ..., 1413, wherein the layers can comprise an intrinsic order introduced by the edges 1440, ..., 1442 between the nodes 1420, ..., 1432. In particular, edges 1440, ..., 1442 can exist only between neighboring layers of nodes. In the displayed embodiment, there is an input layer 1410 comprising only nodes 1420, ..., 1422 without an incoming edge, an output layer 1413 comprising only nodes 1431, 1432 without outgoing edges, and hidden layers 1411, 1412 in-between the input layer 1410 and the output layer 1413. In general, the number of hidden layers 1411, 1412 can be chosen arbitrarily. The number of nodes 1420, ..., 1422 within the input layer 1410 usually relates to the number of input values of the neural network, and the number of nodes 1431, 1432 within the output layer 1413 usually relates to the number of output values of the neural network.

In particular, a (real) number can be assigned as a value to every node 1420, ..., 1432 of the neural network 1400. Here, $x^{(n)}_i$ denotes the value of the i-th node 1420, ..., 1432 of the n-th layer 1410, ..., 1413. The values of the nodes 1420, ..., 1422 of the input layer 1410 are equivalent to the input values of the neural network 1400, the values of the nodes 1431, 1432 of the output layer 1413 are equivalent to the output value of the neural network 1400. Furthermore, each edge 1440, ..., 1442 can comprise a weight being a real number, in particular, the weight is a real number within the interval [-1, 1] or within the interval [0, 1]. Here, $w^{(m,n)}_{i,j}$ denotes the weight of the edge between the i-th node 1420, ..., 1432 of the m-th layer 1410, ..., 1413 and the j-th node 1420, ..., 1432 of the n-th layer 1410, ..., 1413. Furthermore, the abbreviation $w^{(n)}_{i,j}$ is defined for the weight $w^{(n,n+1)}_{i,j}$.

In particular, to calculate the output values of the neural network 1400, the input values are propagated through the neural network. In particular, the values of the nodes 1420, ..., 1432 of the (n+1)-th layer 1410, ..., 1413 can be calculated based on the values of the nodes 1420, ..., 1432 of the n-th layer 1410, ..., 1413 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 1410 are given by the input of the neural network 1400, wherein values of the first hid-den layer 1411 can be calculated based on the values of the input layer 1410 of the neural network, wherein values of the second hidden layer 1412 can be calculated based in the values of the first hidden layer 1411, etc.

In order to set the values $w^{(m,n)}_{i,j}$ for the edges, the neural network 1400 has to be trained using training data. In particular, training data comprises training input data and training output data (denoted as $t_i$). For a training step, the neural network 1400 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 1400 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)_j} \;=\; \left( \sum_k \delta^{(n+1)_k} \cdot w^{(n+1)_{j,k}} \right) \cdot f' \left( \sum_i x^{(n)_i} \cdot w^{(n)_{i,j}} \right)$$

based on $\delta^{(n+1)}{}_j$, if the (n+1)-th layer is not the output layer, and

$$\delta^{(n)_j} \;=\; \left( x^{(n+1)_j} - t^{(n+1)_j} \right) \cdot f' \left( x^{(n)_i} \cdot w^{(n)_{i,j}} \right)$$

if the (n+1)-th layer is the output layer 1413, wherein f is the first derivative of the activation function, and $t^{(n+1)}{}_j$ is the comparison training value for the j-th node of the output layer 1413.

A convolutional neural network is a neural network that uses a convolution operation instead of general matrix multiplication in at least one of its layers (so-called "convolutional layer"). In particular, a convolutional layer performs a dot product of one or more convolution kernels with the convolutional layer's input data / image, wherein the entries of the one or more convolution kernels are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

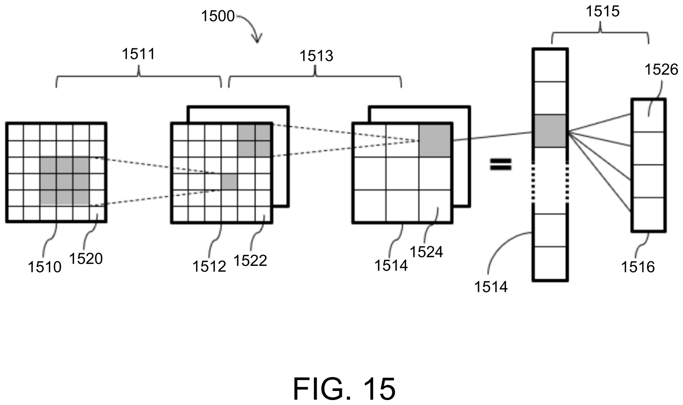

Figure 15 shows an embodiment of a convolutional neural network 1500 that may be used to implement one or more machine learning models described herein. In the displayed embodiment, the convolutional neural network 1500 comprises an input node layer 1510, a convolutional layer 1511, a pooling layer 1513, a fully connected layer 1514 and an output node layer 1516, as well as hidden node layers 1512, 1514. Alternatively, the convolutional neural network 1500 can comprise several convolutional layers 1511, several pooling layers 1513 and several fully connected layers 1515, as well as other types of layers. The order of the layers can be chosen arbitrarily, usually fully connected layers 1515 are used as the last layers before the output layer 1516.

In particular, within a convolutional neural network 1500 nodes 1520, 1522, 1524 of a node layer 1510, 1512, 1514 can be considered to be arranged as a d-dimensional matrix or as a d-dimensional image. In particular, in the two-dimensional case the value of the node 1520, 1522, 1524 indexed with i and j in the n-th node layer 1510, 1512, 1514 can be denoted as x(n)[i, j]. However, the arrangement of the nodes 1520, 1522, 1524 of one node layer 1510, 1512, 1514 does not have an effect on the calculations executed within the convolutional neural network 1500 as such, since these are given solely by the structure and the weights of the edges.

A convolutional layer 1511 is a connection layer between an anterior node layer 1510 (with node values x(n-1)) and a posterior node layer 1512 (with node values x(n)). In particular, a convolutional layer 1511 is characterized by the structure and the weights of the incoming edges forming a convolution operation based on a certain number of kernels. In particular, the structure and the weights of the edges of the convolutional layer 1511 are chosen such that the values x(n) of the nodes 1522 of the posterior node layer 1512 are calculated as a convolution x(n) = K * x(n-1) based on the values x(n-1) of the nodes 1520 anterior node layer 1510, where the convolution * is defined in the two-dimensional case as

$$x_k^{(n)}[i,j] = \left( K \; {}^*x^{(n\text{-}1)} \right)[i,j] = \sum_{i'} \sum_{j'} K \; [i',j'] \cdot x^{(n\text{-}1)}[i\text{-}i', \; j\text{-}j'].$$

Here the kernel K is a d-dimensional matrix (in this embodiment, a two-dimensional matrix), which is usually small compared to the number of nodes 1520, 1522 (e.g., a 3x3 matrix, or a 5x5 matrix). In particular, this implies that the weights of the edges in the convolution layer 1511 are not independent, but chosen such that they produce said convolution equation. In particular, for a kernel being a 3x3 matrix, there are only 9 independent weights (each entry of the kernel matrix corresponding to one independent weight), irrespectively of the number of nodes 1520, 1522 in the anterior node layer 1510 and the posterior node layer 1512.

In general, convolutional neural networks 1500 use node layers 1510, 1512, 1514 with a plurality of channels, in particular, due to the use of a plurality of kernels in convolutional layers 1511. In those cases, the node layers can be considered as (d+1)-dimensional matrices (the first dimension indexing the channels). The action of a convolutional layer 1511 is then a two-dimensional example defined as

$$x^{(n)_b}[i,j] = \sum_a K_{a,b} * x^{(n-1)_a}[i,j] = \sum_a \sum_{i'} \sum_{j'} K_{a,b}[i',j'] \cdot x^{(n-1)_a}[i-i', j-j']$$

where $x^{(n-1)_a}$ corresponds to the a-th channel of the anterior node layer 1510, $x^{(n)_b}$ corresponds to the b-th channel of the posterior node layer 1512 and $K_{a,b}$ corresponds to one of the kernels. If a convolutional layer 1511 acts on an anterior node layer 1510 with A channels and outputs a posterior node layer 1512 with B channels, there are A·B independent d-dimensional kernels $K_{a,b}$.

In general, in convolutional neural networks 1500 activation functions are used. In this embodiment ReLU (acronym for "Rectified Linear Units") is used, with $R(z) = \max(0, z)$, so that the action of the convolutional layer 1511 in the two-dimensional example is

$$x^{(n)_b}[i,j] = R\left( \sum_a \left( K_{a,b} * x^{(n-1)a} \right)[i,j] \right)$$

$$= R\left( \sum_a \sum_{i'} \sum_{j'} K_{a,b}[i',j'] \cdot x^{(n-1)a}[i-i', j-j'] \right)$$

It is also possible to use other activation functions, e.g., ELU (acronym for "Exponential Linear Unit"), LeakyReLU, Sigmoid, Tanh or Softmax.

In the displayed embodiment, the input layer 1510 comprises 36 nodes 1520, arranged as a two-dimensional 6x6 matrix. The first hidden node layer 1512 comprises 72 nodes 1522, arranged as two two-dimensional 6x6 matrices, each of the two matrices being the result of a convolution of the values of the input layer with a 3x3 kernel within the convolutional layer 1511. Equivalently, the nodes 1522 of the first hidden node layer 1512 can be interpreted as arranged as a three-dimensional 2x6x6 matrix, wherein the first dimension correspond to the channel dimension.

The advantage of using convolutional layers 1511 is that spatially local correlation of the input data can exploited by enforcing a local connectivity pattern between nodes of adjacent layers, in particular by each node being connected to only a small region of the nodes of the preceding layer.

A pooling layer 1513 is a connection layer between an anterior node layer 1512 (with node values x(n-1)) and a posterior node layer 1514 (with node values x(n)). In particular, a pooling layer 1513 can be characterized by the structure and the weights of the edges and the activation function forming a pooling operation based on a non-linear pooling function f. For example, in the two-dimensional case the values x(n) of the nodes 1524 of the posterior node layer 1514 can be calculated based on the values x(n-1) of the nodes 1522 of the anterior node layer 1512 as

$$x^{(n)_b}[i,j] = f(x^{(n-1)}[id_1, jd_2], ..., x^{(n-1)_b}[(i+1)d_1 \text{-} 1, (j+1)d_2 \text{-} 1])$$

In other words, by using a pooling layer 1513 the number of nodes 1522, 1524 can be reduced, by re-placing a number d1 ·d2 of neighboring nodes 1522 in the anterior node layer 1512 with a single node 1522 in the posterior node layer 1514 being calculated as a function of the values of said number of neighboring nodes. In particular, the pooling function f can be the max-function, the average or the L2-Norm. In particular, for a pooling layer 1513 the weights of the incoming edges are fixed and are not modified by training.

The advantage of using a pooling layer 1513 is that the number of nodes 1522, 1524 and the number of parameters is reduced. This leads to the amount of computation in the network being reduced and to a control of overfitting.

In the displayed embodiment, the pooling layer 1513 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 1500 are fully connected layers 1515. A fully connected layer 1515 is a connection layer between an anterior node layer 1514 and a posterior node layer 1516. A fully connected layer 1513 can be characterized by the fact that a majority, in particular, all edges between nodes 1514 of the anterior node layer 1514 and the nodes 1516 of the posterior node layer are present, and wherein the weight of each of these edges can be adjusted individually.

In this embodiment, the nodes 1524 of the anterior node layer 1514 of the fully connected layer 1515 are displayed both as two-dimensional matrices, and additionally as non-related nodes (indicated as a line of nodes, wherein the number of nodes was reduced for a better presentability). This operation is also denoted as "flattening". In this embodiment, the number of nodes 1526 in the posterior node layer 1516 of the fully connected layer 1515 smaller than the number of nodes 1524 in the anterior node layer 1514. Alternatively, the number of nodes 1526 can be equal or larger.

Furthermore, in this embodiment the Softmax activation function is used within the fully connected layer 1515. By applying the Softmax function, the sum the values of all nodes 1526 of the output layer 1516 is 1, and all values of all nodes 1526 of the output layer 1516 are real numbers between 0 and 1. In particular, if using the convolutional neural network 1500 for categorizing input data, the values of the output layer 1516 can be interpreted as the probability of the input data falling into one of the different categories.

In particular, convolutional neural networks 1500 can be trained based on the backpropagation algorithm. For preventing overfitting, methods of regularization can be used, e.g., dropout of nodes 1520, ..., 1524, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

A generative adversarial model (an acronym is GA model) comprises a generative function and a discriminative function, wherein the generative function creates synthetic data, and the discriminative function distinguishes between synthetic and real data. By training the generative function and/or the discriminative function on the one hand the generative function is configured to create synthetic data which is incorrectly classified by the discriminative function as real, on the other hand the discriminative function is configured to distinguish between real data and synthetic data generated by the generative function. In the notion of game theory, a generative adversarial model can be interpreted as a zero-sum game. The training of the generative function and/or of the discriminative function is based, in particular, on the minimization of a cost function.

By using a GA model, based on a set of training data synthetic data can be generated that has the same characteristics as the training data set. The training of the GA model can be based on data not being annotated (unsupervised learning), so that there is low effort in training a GA model.

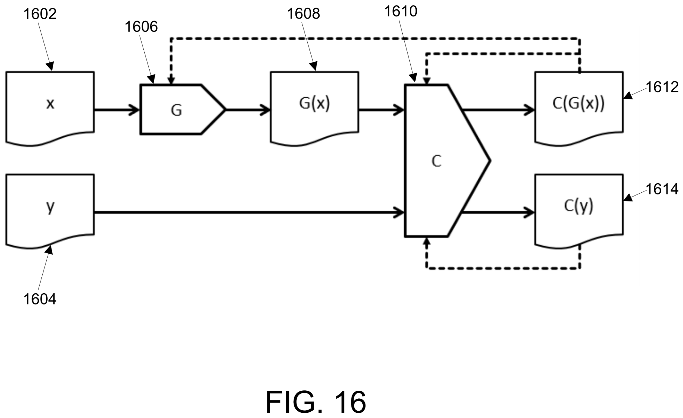

Figure 16 shows a data flow diagram according to an embodiment for using a generative adversarial network for creating synthetic output data $G(x)$ 1608 based on input data $x$ 1602 that is indistinguishable from real output data $y$ 1604, in accordance with one or more embodiments. The synthetic output data $G(x)$ 1608 has the same structure as the real output data $y$ 1604, but its content is not derived from real world data.

The generative adversarial network comprises a generator function G 1606 and a classifier function C 1610 which are trained jointly. The task of the generator function G 1606 is to provide realistic synthetic output data $G(x)$ 1608 based on input data $x$ 1602, and the task of the classifier function C 1610 is to distinguish between real output data $y$ 1604 and synthetic output data $G(x)$ 1608. In particular, the output of the classifier function C 1610 is a real number between 0 and 1 corresponding to the probability of the input value being real data, so that an ideal classifier function would calculate an output value of $C(y)$ 1614 $\approx$ 1 for real data $y$ 1604 and $C(G(x))$ 1612 $\approx$ 0 for synthetic data $G(x)$ 1608.

Within the training process, parameters of the generator function G 1606 are adapted so that the synthetic output data $G(x)$ 1608 has the same characteristics as real output data $y$ 1604, so that the classifier function C 1610 cannot distinguish between real and synthetic data anymore. At the same time, parameters of the classifier function C 1610 are adapted so that it distinguishes between real and synthetic data in the best possible way. Here, the training relies on pairs comprising input data $x$ 1602 and the corresponding real output data $y$ 1604. Within a single training step, the generator function G 1606 is applied to the input data $x$ 1602 for generating synthetic output data $G(x)$ 1608. Furthermore, the classifier function C 1610 is applied to the real output data $y$ 1604 for generating a first classification result $C(y)$ 1614. Additionally, the classifier function C 1610 is applied to the synthetic output data $G(x)$ 1608 for generating a second classification result $C(G(x))$ 1612.

Adapting the parameters of the generative function G 1606 and the classifier function C 1610 is based on minimizing a cost function by using the backpropagation algorithm, respectively. In this embodiment, the cost function Kc for the classifier function C 1610 is $K_C \propto$ - BCE($C(y)$, 1) - BCE($C(G(x))$, 0), wherein BCE denotes the binary cross entropy defined as BCE$(z, z') = z' \cdot \log(z) + (1 - z') \cdot \log(1 - z)$. By using this cost function, both wrongly classifying real output data as synthetic (indicated by $C(y) \approx 0$) and wrongly classifying synthetic output data as real (indicated as $C(G(x))$ 1612 $\approx$ 1) increases the cost function Kc to be minimized. Furthermore, the cost function $K_G$ for the generator function G 1606 is $K_G$

$\propto$-BCE(C(G(x)), 1) = - log (C(G(x))). By using this cost function, correctly classified synthetic output data (indicated as C(G(x)) 1612 $\approx$ 0) leads to an increase of the cost function $K_G$ to be minimized.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

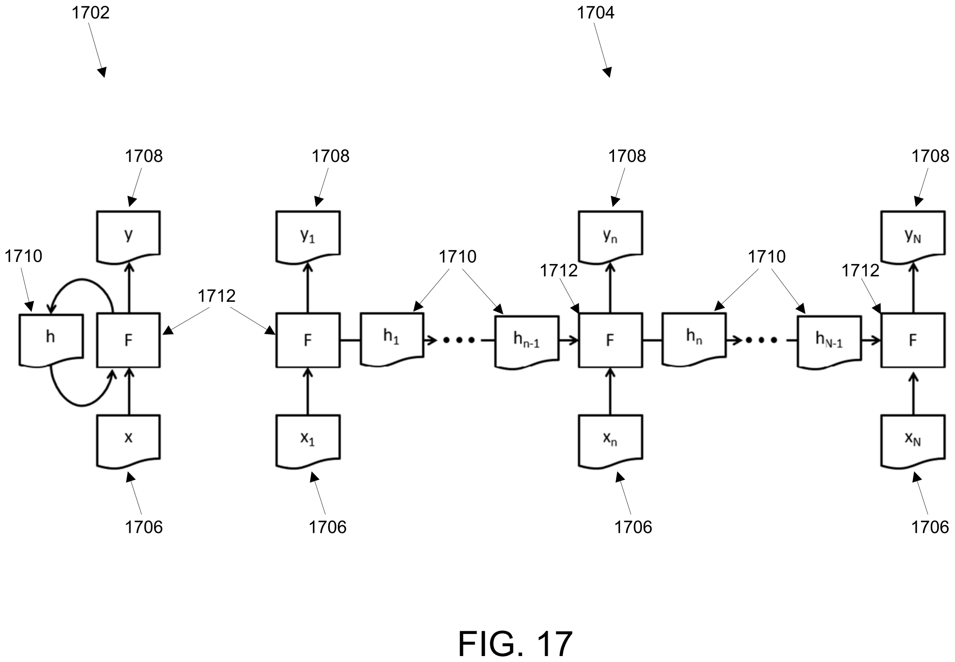

Figure 17 shows the schematic structure of a recurrent machine learning model F, both in a recurrent representation 1702 and in an unfolded representation 1704, that may be used to implement one or more machine learning models described herein. The recurrent machine learning model takes as input several input datasets x, $x_1$, ..., $x_N$ 1706 and creates a corresponding set of output datasets y, $y_1$, ..., $y_N$ 1708. Furthermore, the output depends on a so-called hidden vector h, h1, ..., $h_N$ 1710, which implicitly comprises information about input datasets previously used as input for the recurrent machine learning model F 1712. By using these hidden vectors h, $h_1$, ..., $h_N$ 1710, a sequentiality of the input datasets can be leveraged.

In a single step of the processing, the recurrent machine learning model F 1712 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 1712 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 1712 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(x_{n-1}, h_{n-2}))$.

All features described above with respect to one of the disclosed methods may also be implemented in the corresponding apparatus or system, or vice versa.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1-2 and 8-12. Certain steps or functions of the methods and workflows described herein, including one or more of the steps or functions of Figures 1-2 and 8-12, may be performed by a server or by another processor in a network-based cloud-computing system. Certain steps or functions of the methods and workflows described herein, including one or more of the steps of Figures 1-2 and 8-12, may be performed by a client computer in a network-based cloud computing system. The steps or functions of the methods and workflows described herein, including one or more of the steps of Figures 1-2 and 8-12, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1-2 and 8-12, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 1802 that may be used to implement systems, apparatuses, and methods described herein is depicted in Figure 18. Computer 1802 includes a processor 1804 operatively coupled to a data storage device 1812 and a memory 1810. Processor 1804 controls the overall operation of computer 1802 by executing computer program instructions that define such operations. The computer program instructions may be stored in data storage device 1812, or other computer readable medium, and loaded into memory 1810 when execution of the computer program instructions is desired. Thus, the method and workflow steps or functions of Figures 1-2 and 8-12 can be defined by the computer program instructions stored in memory 1810 and/or data storage device 1812 and controlled by processor 1804 executing the computer program instructions. For example, the computer program instructions can be implemented as computer executable code programmed by one skilled in the art to perform the method and workflow steps or functions of Figures 1-2 and 8-12. Accordingly, by executing the computer program instructions, the processor 1804 executes the method and workflow steps or functions of Figures 1-2 and 8-12. Computer 1802 may also include one or more network interfaces 1806 for communicating with other devices via a network. Computer 1802 may also include one or more input/output devices 1808 that enable user interaction with computer 1802 (e.g., display, keyboard, mouse, speakers, buttons, etc.).

Processor 1804 may include both general and special purpose microprocessors, and may be the sole processor or one of multiple processors of computer 1802. Processor 1804 may include one or more central processing units (CPUs), for example. Processor 1804, data storage device 1812, and/or memory 1810 may include, be supplemented by, or incorporated in, one or more application-specific integrated circuits (ASICs) and/or one or more field programmable gate arrays (FPGAs).

Data storage device 1812 and memory 1810 each include a tangible non-transitory computer readable storage medium. Data storage device 1812, and memory 1810, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 1808 may include peripherals, such as a printer, scanner, display screen, etc. For example, input/output devices 1808 may include a display device such as a cathode ray tube (CRT) or liquid crystal display (LCD) monitor for displaying information to the user, a keyboard, and a pointing device such as a mouse or a trackball by which the user can provide input to computer 1802.

An image acquisition device 1814 can be connected to the computer 1802 to input image data (e.g., medical images) to the computer 1802. It is possible to implement the image acquisition device 1814 and the computer 1802 as one device. It is also possible that the image acquisition device 1814 and the computer 1802 communicate wirelessly through a network. In a possible embodiment, the computer 1802 can be located remotely with respect to the image acquisition device 1814.

Any or all of the systems, apparatuses, and methods discussed herein may be implemented using one or more computers such as computer 1802.

One skilled in the art will recognize that an implementation of an actual computer or computer system may have other structures and may contain other components as well, and that Figure 18 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention as defined .

The following is a list of non-limiting illustrative embodiments disclosed herein:

Illustrative embodiment 1. A computer-implemented method comprising: receiving one or more medical images; segmenting one or more anatomical objects from the one or more medical images; determining radiotherapy configurations for one or more objectives based on the one or more medical images and the segmentations of the one or more anatomical objectives; generating a candidate radiotherapy plan based on the radiotherapy configurations; performing a quality assessment of the candidate radiotherapy plan; updating the one or more objectives based on results of the quality assessment and repeating the determining, the generating, and the performing for one or more iterations using the updated one or more objectives as the one or more objectives to generate the candidate radiotherapy plan for a last iteration as a final radiotherapy plan; and outputting the final radiotherapy plan.

Illustrative embodiment 2. The computer-implemented method of illustrative embodiment 1, wherein performing a quality assessment of the candidate radiotherapy plan comprises: calculating a score for each of a plurality of metrics for the candidate radiotherapy plan; and combining the scores to generate a final score of the candidate radiotherapy plan.

Illustrative embodiment 3. The computer-implemented method of any of illustrative embodiments 1-2, wherein repeating the determining, the generating, and the performing for one or more iterations using the updated one or more objectives as the one or more objectives to generate the candidate radiotherapy plan for a last iteration as a final radiotherapy plan comprises: comparing the results of the quality assessment with results of a quality assessment of a preceding iteration; determining whether the comparison satisfies a threshold; and in response to determining that the comparison does not satisfy the threshold, repeating the determining, the generating, and the performing.

Illustrative embodiment 4. The computer-implemented method of any of illustrative embodiments 1-3, wherein updating the one or more objectives based on results of the quality assessment comprises: adding one or more metrics resulting from the quality assessment of the candidate radiotherapy plan to the one or more objectives.

Illustrative embodiment 5. The computer-implemented method of any of illustrative embodiments 1-4, wherein updating the one or more objectives based on results of the quality assessment comprises: adding a margin to at least one of the one or more objectives based on the results of the quality assessment of the candidate radiotherapy plan.

Illustrative embodiment 6. The computer-implemented method of any of illustrative embodiments 1-5, further comprising generating PTV (planning target view) helper structures based on the one or more medical images, wherein determining radiotherapy configurations for one or more objectives based on the one or more medical images and the segmentations of the one or more anatomical objectives comprises: determining the radiotherapy configurations for the one or more objectives further based on the PTV helpers.

Illustrative embodiment 7. The computer-implemented method of any of illustrative embodiments 1-6, wherein the radiotherapy configurations comprise beam configuration and dose.

Illustrative embodiment 8. The computer-implemented method of any of illustrative embodiments 1-7, wherein the radiotherapy configurations comprise a maximum and minimum dose and dose volume constraints.

Illustrative embodiment 9. The computer-implemented method of any of illustrative embodiments 1-8, further comprising: training an AI (artificial intelligence) model based on the final radiotherapy plan.

Illustrative embodiment 10. An apparatus comprising: means for receiving one or more medical images; means for segmenting one or more anatomical objects from the one or more medical images; means for determining radiotherapy configurations for one or more objectives based on the one or more medical images and the segmentations of the one or more anatomical objectives; means for generating a candidate radiotherapy plan based on the radiotherapy configurations; means for performing a quality assessment of the candidate radiotherapy plan; means for updating the one or more objectives based on results of the quality assessment and means for repeating the determining, the generating, and the performing for one or more iterations using the updated one or more objectives as the one or more objectives to generate the candidate radiotherapy plan for a last iteration as a final radiotherapy plan; and means for outputting the final radiotherapy plan.

Illustrative embodiment 11. The apparatus of illustrative embodiment 10, wherein the means for performing a quality assessment of the candidate radiotherapy plan comprises: means for calculating a score for each of a plurality of metrics for the candidate radiotherapy plan; and means for combining the scores to generate a final score of the candidate radiotherapy plan.

Illustrative embodiment 12. The apparatus of any of illustrative embodiments 10-11, wherein the means for repeating the determining, the generating, and the performing for one or more iterations using the updated one or more objectives as the one or more objectives to generate the candidate radiotherapy plan for a last iteration as a final radiotherapy plan comprises: means for comparing the results of the quality assessment with results of a quality assessment of a preceding iteration; means for determining whether the comparison satisfies a threshold; and in response to determining that the comparison does not satisfy the threshold, means for repeating the determining, the generating, and the performing.

Illustrative embodiment 13. The apparatus of any of illustrative embodiments 10-12, wherein the means for updating the one or more objectives based on results of the quality assessment comprises: means for adding one or more metrics resulting from the quality assessment of the candidate radiotherapy plan to the one or more objectives.

Illustrative embodiment 14. The apparatus of any of illustrative embodiments 10-13, wherein the means for updating the one or more objectives based on results of the quality assessment comprises: means for adding a margin to at least one of the one or more objectives based on the results of the quality assessment of the candidate radiotherapy plan.

Illustrative embodiment 15. A non-transitory computer-readable storage medium comprising instructions which, when executed by a computer, cause the computer to carry out operations comprising: receiving one or more medical images; segmenting one or more anatomical objects from the one or more medical images; determining radiotherapy configurations for one or more objectives based on the one or more medical images and the segmentations of the one or more anatomical objectives; generating a candidate radiotherapy plan based on the radiotherapy configurations; performing a quality assessment of the candidate radiotherapy plan; updating the one or more objectives based on results of the quality assessment and repeating the determining, the generating, and the performing for one or more iterations using the updated one or more objectives as the one or more objectives to generate the candidate radiotherapy plan for a last iteration as a final radiotherapy plan; and outputting the final radiotherapy plan.

Illustrative embodiment 16. The non-transitory computer-readable storage medium of illustrative embodiment 15, wherein performing a quality assessment of the candidate radiotherapy plan comprises: calculating a score for each of a plurality of metrics for the candidate radiotherapy plan; and combining the scores to generate a final score of the candidate radiotherapy plan.

Illustrative embodiment 17. The non-transitory computer-readable storage medium of any of illustrative embodiments 15-16, the operations further comprising generating PTV (planning target view) helper structures based on the one or more medical images, wherein determining radiotherapy configurations for one or more objectives based on the one or more medical images and the segmentations of the one or more anatomical objectives comprises: determining the radiotherapy configurations for the one or more objectives further based on the PTV helpers.

Illustrative embodiment 18. The non-transitory computer-readable storage medium of any of illustrative embodiments 15-17, wherein the radiotherapy configurations comprise beam configuration and dose.

Illustrative embodiment 19. The non-transitory computer-readable storage medium of any of illustrative embodiments 15-18, wherein the radiotherapy configurations comprise a maximum and minimum dose and dose volume constraints.

Illustrative embodiment 20. The non-transitory computer-readable storage medium of any of illustrative embodiments 15-19, the operations further comprising: training an AI (artificial intelligence) model based on the final radiotherapy plan.

**Claims**

1. A computer-implemented method comprising:

   receiving (102) one or more medical images;
   segmenting (104, 202) one or more anatomical objects from the one or more medical images;
   determining (106, 204) radiotherapy configurations for one or more objectives based on the one or more medical images and the segmentations of the one or more anatomical objectives;
   generating (108, 206) a candidate radiotherapy plan based on the radiotherapy configurations;
   performing (110, 208) a quality assessment of the candidate radiotherapy plan;
   updating (112, 210) the one or more objectives based on results of the quality assessment and repeating the determining, the generating, and the performing for one or more iterations using the updated one or more objectives as the one or more objectives to generate the candidate radiotherapy plan for a last iteration as a final radiotherapy plan (700); and
   outputting (114) the final radiotherapy plan.

2. The computer-implemented method of claim 1, wherein performing a quality assessment of the candidate radiotherapy plan comprises:

calculating a score for each of a plurality of metrics for the candidate radiotherapy plan; and
combining the scores to generate a final score of the candidate radiotherapy plan.

3. The computer-implemented method of any of claims 1 - 2, wherein repeating the determining, the generating, and the performing for one or more iterations using the updated one or more objectives as the one or more objectives to generate the candidate radiotherapy plan for a last iteration as a final radiotherapy plan comprises:

comparing the results of the quality assessment with results of a quality assessment of a preceding iteration;
determining whether the comparison satisfies a threshold; and
in response to determining that the comparison does not satisfy the threshold, repeating the determining, the generating, and the performing.

4. The computer-implemented method of any of claims 1 - 3, wherein updating the one or more objectives based on results of the quality assessment comprises:
adding one or more metrics resulting from the quality assessment of the candidate radiotherapy plan to the one or more objectives.

5. The computer-implemented method of any of claims 1 - 4, wherein updating the one or more objectives based on results of the quality assessment comprises:
adding a margin to at least one of the one or more objectives based on the results of the quality assessment of the candidate radiotherapy plan.

6. The computer-implemented method of any of claims 1 - 5, further comprising generating planning target view, PTV, helper structures based on the one or more medical images, wherein determining radiotherapy configurations for one or more objectives based on the one or more medical images and the segmentations of the one or more anatomical objectives comprises:
determining the radiotherapy configurations for the one or more objectives further based on the PTV helpers.

7. The computer-implemented method of any of claims 1 - 6, wherein the radiotherapy configurations comprise beam configuration and dose.

8. The computer-implemented method of any of claims 1 - 7, wherein the radiotherapy configurations comprise a maximum and minimum dose and dose volume constraints.

9. The computer-implemented method of any of claims 1 - 8, further comprising:
training an artificial intelligence, AI, model based on the final radiotherapy plan.

10. An apparatus comprising:

means for receiving (102) one or more medical images;
means for segmenting (104, 202) one or more anatomical objects from the one or more medical images;
means for determining (106, 204) radiotherapy configurations for one or more objectives based on the one or more medical images and the segmentations of the one or more anatomical objectives;
means for generating (108, 206) a candidate radiotherapy plan based on the radiotherapy configurations;
means for performing (110, 208) a quality assessment of the candidate radiotherapy plan;
means for updating (112, 210) the one or more objectives based on results of the quality assessment and repeating the determining, the generating, and the performing for one or more iterations using the updated one or more objectives as the one or more objectives to generate the candidate radiotherapy plan for a last iteration as a final radiotherapy plan (700); and
means for outputting (114) the final radiotherapy plan.

11. The apparatus of claim 10, wherein the means for performing a quality assessment of the candidate radiotherapy plan comprises:

means for calculating a score for each of a plurality of metrics for the candidate radiotherapy plan; and
means for combining the scores to generate a final score of the candidate radiotherapy plan.

**12.** The apparatus of any of claims 10 - 11, wherein the means for repeating the determining, the generating, and the performing for one or more iterations using the updated one or more objectives as the one or more objectives to generate the candidate radiotherapy plan for a last iteration as a final radiotherapy plan comprises:

means for comparing the results of the quality assessment with results of a quality assessment of a preceding iteration;
means for determining whether the comparison satisfies a threshold; and
in response to determining that the comparison does not satisfy the threshold, means for repeating the determining, the generating, and the performing.

**13.** The apparatus of any of claims 10 - 12, wherein the means for updating the one or more objectives based on results of the quality assessment comprises:
means for adding one or more metrics resulting from the quality assessment of the candidate radiotherapy plan to the one or more objectives.

**14.** The apparatus of any of claims 10 - 13, wherein the means for updating the one or more objectives based on results of the quality assessment comprises:
means for adding a margin to at least one of the one or more objectives based on the results of the quality assessment of the candidate radiotherapy plan.

**15.** A non-transitory computer-readable storage medium comprising instructions which, when executed by a computer, cause the computer to carry out operations of the computer-implemented method according to any of claims 1 - 9.

# FIG. 1

<u>100</u>

```
┌─────────────────────────────────────────────────────┐
│         Receive one or more medical images          │
│                        102                          │
└─────────────────────────────────────────────────────┘
                          │
                          ▼
┌─────────────────────────────────────────────────────┐
│  Segment one or more anatomical objects from the     │
│            one or more medical images                │
│                        104                          │
└─────────────────────────────────────────────────────┘
                          │
                          ▼
┌─────────────────────────────────────────────────────┐
│  Determine radiotherapy configurations for one or    │
│  more objectives based on the one or more medical    │
│  images and the segmentations of the one or          │
│           more anatomical objects                    │
│                        106                          │
└─────────────────────────────────────────────────────┘
                          │
                          ▼
┌─────────────────────────────────────────────────────┐
│  Generate a candidate radiotherapy plan based on     │
│           the radiotherapy configurations            │
│                        108                          │
└─────────────────────────────────────────────────────┘
                          │
                          ▼
┌─────────────────────────────────────────────────────┐
│  Perform a quality assessment of the candidate       │
│              radiotherapy plan                       │
│                        110                          │
└─────────────────────────────────────────────────────┘
                          │
                          ▼
┌─────────────────────────────────────────────────────┐
│  Update the one or more objectives based on results  │
│  of the quality assessment and repeating steps 106,  │
│  108, and 110 for one or more iterations using the   │
│  updated radiotherapy objectives and the radiotherapy│
│  objectives to generate the candidate radiotherapy   │
│  plan for a last iteration as a final radiotherapy   │
│  plan                                                │
│                        112                          │
└─────────────────────────────────────────────────────┘
                          │
                          ▼
┌─────────────────────────────────────────────────────┐
│         Output the final radiotherapy plan           │
│                        114                          │
└─────────────────────────────────────────────────────┘
```

200

202  204  206  208

AI Contouring  +  Clinical PTVs  +  Helper Structs  +  RapidPlan

Configuration / Objectives

Optimization & Calculation

Quality Assessment

Adjust Objectives

210

FIG. 2

EP 4 775 259 A1

| PTV & Helpers | Comments | PTV & Helpers | Comments |
|---|---|---|---|
| PTVHigh | PTV with the largest prescription | RingPTVHigh | [(PTVHigh + 30mm) − (PTVHigh + 2mm)] AND Body |
| PTVHighOPT | PTVHigh − (BrachialPlexus + 2mm) | | |
| PTVMid | PTV with the middle prescriptions, and include the PTVHigh | RingPTVMid | [(PTVMid + 30mm) − (PTVMid + 2mm) - (PTVHigh + 6mm)] AND Body |
| PTVMidOPT | PTVMid − (PTVHigh + 3mm) | PTVMid-PTVHigh | as the structure name |
| PTVLow | PTV with the lowest prescription, and include the PTVMid | RingPTVLow | [(PTVLow + 30mm) − (PTVLow + 2mm) - (PTVMid + 6mm) - (PTVHigh + 9mm)] AND Body |
| PTVLowOPT | PTVLow - (PTVMid + 3mm ) - (PTVHigh + 6mm) | PTVLow-PTVMid | as the structure name |
| PTVTotal | union of all PTVs | | |

| OARs | OARs | OARs | OARs-PTV |
|---|---|---|---|
| BrachiaPlexus | Larynx | Posterior_Neck | Larynx-PTV |
| Brain | Lens_L | Shoulders | Mandible-PTV |
| BrainStem | Lens_R | SpinalCord | OCavity-PTV |
| BrainStem_03 | Lips | SpinalCord_05 | ParotdCon-PTV |
| Chiasm | OpticNerve_L | Submandibular | ParotdIps-PTV |
| Cochlea_L | OpticNerve_R | Trachea | Parotids-PTV |
| Cochlea_R | OralCavity | Lungs | PharCost-PTV |
| Esophagus | Parotids | Mandible | Submand-PTV |
| Eyes | PharynxConst | Body | SubmandL-PTV |
| LacrimalGlands | Pituitary | TMJoint | SubmandR-PTV |

FIG. 3

# FIG. 4

400

| ROI | Type | Dose | Volume | Operation | Prior |
|---|---|---|---|---|---|
| PTV | Point | 60 Gy | 100 % | Lower | 300 |
| PTV | Point | 63 Gy | 0 % | Upper | 300 |
| BodyRing | Point | 63 Gy | 0 % | Upper | 250 |
| TotalLung-GTV | Point | 20 Gy | 18.5 % | Upper | 100 |
| TotalLung-GTV | Point | 5 Gy | 32.5 % | Upper | 100 |
| TotalLung-GTV | Mean | 5 Gy | N/A | Upper | 100 |
| SpinalCord | Point | 22 Gy | 0 % | Upper | 80 |
| Heart | Point | 55 Gy | 0 % | Upper | 60 |
| Heart | Mean | 5 Gy | N/A | Upper | 60 |
| LAD | Point | 55 Gy | 0 % | Upper | 60 |
| Esophagus | Mean | 15 Gy | N/A | Upper | 60 |
| BrachPlexus | Mean | 15 Gy | N/A | Upper | 60 |
| GreatVessels | Mean | 55 Gy | 0 % | Upper | 60 |
| Trachea | Mean | 55 Gy | 0 % | Upper | 60 |
| RingPTV | Mean | 67 Gy | 0 % | Upper | 60 |

| ROI | Metric Type | Input | ROI | Metric Type | Input | ROI | Metric Type | Input |
|---|---|---|---|---|---|---|---|---|
| PTVHighOPT | VolAtDose | 70.0 Gy | PTVHighOPT | DoseAtVol | 99.5 % | PTVHighOPT | DoseAtVol | 0.03 cc |
| PTVMid | VolAtDose | 63.0 Gy | PTVMid | DoseAtVol | 99.5 % | PTVMid-PTVHigh | VolAtDose | 66.2 Gy |
| PTV56 | VolAtDose | 56.0 Gy | PTV56 | DoseAtVol | 99.5 % | PTV56-PTV63 | VolAtDose | 58.8 Gy |
| SpinalCord05 | DoseAtVol | 0.03 cc | SpinalCord05 | VolAtDose | 40.0 Gy | SpinalCord05 | VolAtDose | 30.0 Gy |
| BrainStem03 | DoseAtVol | 0.03 cc | Brain | DoseAtVol | 0.03 cc | Brain | VolAtDose | 50.0 Gy |
| Pituitary | MeanDose | N/A | Chiasm | DoseAtVol | 0.03 cc | OpticNerveL | DoseAtVol | 0.03 cc |
| OpticNerveR | DoseAtVol | 0.03 cc | LacrimalGlands | MeanDose | N/A | CochleaR | VolAtDose | 40.0 Gy |
| CochleaL | VolAtDose | 40.0 Gy | LensR | DoseAtVol | 0.03 cc | LensL | DoseAtVol | 0.03 cc |
| EyeR | DoseAtVol | 0.03 cc | EyeR | MeanDose | N/A | EyeL | DoseAtVol | 0.03 cc |
| Mandible-PTV | VolAtDose | 70.0 Gy | Mandible-PTV | VolAtDose | 60.0 Gy | Mandible-PTV | VolAtDose | 50.0 Gy |
| Esophagus | MeanDose | N/A | Esophagus | DoseAtVol | 0.03 cc | OCavity-PTV | MeanDose | N/A |
| OCavity-PTV | DoseAtVol | 0.03 cc | Larynx-PTV | MeanDose | N/A | Thyroid-PTV | MeanDose | N/A |
| BrachPlexusL | DoseAtVol | 0.1 cc | BrachPlexusR | DoseAtVol | 0.1 cc | SubmandL-PTV | MeanDose | N/A |
| SubmandR-PTV | MeanDose | N/A | TMJoint | DoseAtVol | 0.03 cc | RingPTV70 | DoseAtVol | 0.03 cc |
| RingPTV63 | DoseAtVol | 0.03 cc | RingPTV56 | DoseAtVol | 0.03 cc | PosteriorNeck | VolAtDose | 35.0 Gy |
| Trachea | MeanDose | N/A | Lungs | VolAtDose | 20.0 Gy | Shoulders | MeanDose | N/A |
| Parotids-PTV | MeanDose | N/A | Submand-PTV | MeanDose | N/A | | | |

FIG. 5

EP 4 775 259 A1

# FIG. 6

600

| ROI | Metric Type | Input |
|---|---|---|
| PTV | VolAtDose | 60 Gy |
| CTV | VolAtDose | 60 Gy |
| PTV | VolAtDose | 57 Gy |
| PTV | VolAtDose | 54 Gy |
| PTV | DoseAtVol | 0.03 cc |
| PTV | DoseAtVol | 99.5 % |
| TotalLung-GTV | VolAtDose | 20 Gy |
| TotalLung-GTV | VolAtDose | 5 Gy |
| TotalLung-GTV | MeanDose | N/A |
| SpinalCord | DoseAtVol | 0.03 cc |
| Heart | DoseAtVol | 0.03 cc |
| Heart | MeanDose | N/A |
| LAD | VolAtDose | 15 Gy |
| Esophagus | DoseAtVol | 0.03 cc |
| Esophagus | MeanDose | N/A |
| BrachPlexus | DoseAtVol | 0.03 cc |
| GreatVessels | DoseAtVol | 0.03 cc |
| Trachea | DoseAtVol | 0.03 cc |

FIG. 7

800

FIG. 8

# FIG. 9

900

---

**Algorithm 1** Iterative Planning Processing

---

**Input:** Patient data with CT, PTV/OAR contours, helper structures, prescribed doses for PTVs; ScoreCard for plan evaluation; beam configurations; RapidPlan for a head-and-neck scenario.
**Output:** A series of plans until the ScoreCard convergence.

1:  Initialize plan based on RapidPlan or ScorePlan items
2:  **for** $n = 1$ to *MAX iteration* **do**
3:      Initialize objective buffer $D$
4:      **for** $s$ in *PTVs and its ring structures* **do**
5:        Set the upper / lower point objectives based prescribed dose
6:        Use the same priorities as in the initial plan
7:      **end for**
8:      Calculate the ScoreCard for current plan
9:      **for** $s$ in *ScoreCard OARs and helper structures* **do**
10:       Calculate the DVH Cumulative Data $f_s(\cdot)$
11:       **for** $v$ in *volume points* $\{1, 10, 20, 30, 40, 60, 80\}$ **do**
12:         Calculate the dose $d = f_s(v)$
13:         Calculate the margin and objective dose and priority based on code 1000 of Fig. 10.
14:         Add the objective to objective buffer.
15:       **end for**
16:       **for** items in *ScoreCard* **do**
17:         Calculate the margin and objective dose and priority based on code 1100 of Fig. 11.
18:         Add the objective to objective buffer.
19:       **end for**
20:      **end for**
21:      Optimize with the current objective buffer.
22:      Update current plan.
23:  **end for**

---

# FIG. 10

1000

```
def Dose2Obj(s, value, volume, score):
    if '-PTV' in s.Id and value > 20:
        res = max(value * 0.95, value - 3)
        weight = 120
    elif value > 63 and '-PTV' not in s.Id:
        res = value - 1
        weight = 80
    elif value < 20 and (volume is not None and volume < 50):
        res = max(value * 0.95, value - 2)
        weight = 80
    elif value < 10:
        res = max(value * 0.95, value - 2)
        weight = 80
    else:
        res = max(value * 0.95, value - 2)
        weight = 120
    return round(res, 2), weight
```

# FIG. 11

1100

```
def scorecard2dose(x, ScorePoints):
    ScorePoints = sorted(ScorePoints, key=lambda p: p['PointX'])

    if x < ScorePoints[0]['PointX']:
        x =  ScorePoints[0]['PointX']
    if x > ScorePoints[len(ScorePoints) - 1]['PointX']:
        x = ScorePoints[len(ScorePoints) - 1]['PointX']

    for i in range(len(ScorePoints) - 1):
        x1, y1 = ScorePoints[i]['PointX'], ScorePoints[i]['Score']
        x2, y2 = ScorePoints[i + 1]['PointX'], ScorePoints[i + 1]['Score']
        assert x1 <= x2
        # Check if x is within the current segment
        if x1 <= x <= x2:
            # Calculate the slope (m) and intercept (b) for the line between (x1, y1) and
                (x2, y2)
            m = (y2 - y1) / (x2 - x1 + 1e-5)
            b = y1 - m * x1
            score = m * x + b
            dose_value = max(x * 0.85, x - 7) # add margin to dose value
            priority = 60 + min(abs(m), 7) * 20 / (1 + np.exp(score / 3))
            # calculate the optimization priority
            return dose_value, priority
```

## 1200

FIG. 12

# FIG. 13

1302

1304

FIG. 14

FIG. 15

FIG. 16

FIG. 17

# FIG. 18

1802

Network interface
1806

I/O
1808

Processor
1804

Storage
1812

Memory
1810

Image Acquisition
Device
1814

Europäisches
Patentamt

European
Patent Office

Office européen
des brevets

## EUROPEAN SEARCH REPORT

**Application Number**

EP 26 15 0119

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/207643 A1 (SUHONEN PAULI MIKAEL [FI] ET AL) 27 June 2024 (2024-06-27) * paragraphs [0040] - [0055], [0064] - [0096] * * figures 2,3 * | 1-15 | INV. A61N5/10 |
| X | US 2013/102830 A1 (OTTO KARL [CA] ET AL) 25 April 2013 (2013-04-25) * paragraphs [0055] - [0060] * * figure 3 * | 1,10,15 | |

**TECHNICAL FIELDS SEARCHED (IPC)**

A61N

The present search report has been drawn up for all claims

| Place of search | Date of completion of the search | Examiner |
|---|---|---|
| Munich | 28 April 2026 | Kretschmann, Jana |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                                               

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 26 15 0119

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2026

| Patent document cited in search report | | | Publication date | Patent family member(s) | | | Publication date |
|---|---|---|---|---|---|---|---|
| US 2024207643 | A1 | | 27-06-2024 | CN | 118230901 | A | 21-06-2024 |
| | | | | EP | 4389206 | A1 | 26-06-2024 |
| | | | | US | 2024207643 | A1 | 27-06-2024 |
| US 2013102830 | A1 | | 25-04-2013 | CN | 103038669 | A | 10-04-2013 |
| | | | | EP | 2585854 | A1 | 01-05-2013 |
| | | | | EP | 3722836 | A1 | 14-10-2020 |
| | | | | JP | 5805757 | B2 | 04-11-2015 |
| | | | | JP | 2013529481 | A | 22-07-2013 |
| | | | | US | 2013102830 | A1 | 25-04-2013 |
| | | | | US | 2016236008 | A1 | 18-08-2016 |
| | | | | US | 2018326222 | A1 | 15-11-2018 |
| | | | | US | 2020368553 | A1 | 26-11-2020 |
| | | | | WO | 2011160235 | A1 | 29-12-2011 |

EPO FORM P0459

For more details about this annex : see Official Journal of the European Patent Office, No. 12/82

## EP 4 775 259 A1

**Patent documents cited in the description**

- US 63742940